# FASCICULE DE BREVET EUROPEEN

(11) **EP 3 598 245 B1**
(45) Date de publication et mention de la délivrance du brevet: **04.09.2024**
(21) Numéro de dépôt: 19185615.2
(22) Date de dépôt: 10.07.2019
(51) Int. Cl.: G04C 10/00, G04G 19/10

(54) **MONTRE CONNECTÉE À REMONTAGE MÉCANIQUE**
ARMBANDUHR, DIE MIT EINEM MECHANISCHEN AUFZIEHMECHANISMUS VERBUNDEN IST
WATCH CONNECTED TO MECHANICAL WINDING

(30) Priorité: 20.07.2018 CH 8952018
(43) Date de publication de la demande: 22.01.2020
(73) Titulaire: Sequent AG, 2300 La Chaux-de-Fonds (CH)
(72) Inventeur: Guhl, Harry, 2051 Basel (CH); Buchmann, Adrian, Henley-on-Thames RG9 1EE (GB)
(74) Mandataire: P&TS SA (AG, Ltd.)

(56) Documents cités:
- EP-A1- 3 190 469
- US-A1- 2016 252 883
- US-A1- 2018 004 169

## Description

### Domaine technique

La présente invention concerne une montre électronique comprenant des capteurs et une interface de communication connectable à un réseau informatique, capable d'afficher des indications déterminées sur la base de données provenant du réseau ou des capteurs.

### Etat de la technique

Les dispositifs portables électroniques sont couramment utilisés et largement connus. Des dispositifs tels que laptops, tablettes, téléphones portables, consoles de jeu et lecteurs de musique sont utilisés dans tous les secteurs de l'industrie et du loisir et offrent une infinité de services intégrant des informations provenant d'une interface réseau vers internet, des capteurs propres, et des instruments de calcul et élaboration de données informatiques.

Des efforts de miniaturisation ont permis la création de dispositifs portables toujours plus petits et pratiques, et on connaît déjà maints exemples de montre-bracelet "connectées" ou "intelligentes" capables d'échanger des éléments d'information sur internet et afficher par exemple des notifications au porteur sur la base de ces informations. Plusieurs interfaces d'échange de données sans fils sont utilisées à ce fin, comprenant, entre autres, les réseaux téléphoniques cellulaires, par exemple suivant GSM, GPRS, UMTS, ou tout autre standard approprié, et les réseaux locaux sans fil (WiFi, Bluetooth^{®}, etc.).

On connaît également des montres ou des dispositifs de poignet, connectés à un réseau ou indépendants, munis de capteurs de toutes sortes, par exemple des capteurs GPS, accéléromètres, etc. et de ressources de calcul arrangées pour fournir des indications de l'activité physique, sportive, ou de l'état de santé, intégrant les renseignements provenant de ces capteurs. Ces montres connectées peuvent utiliser n'importe quel système de communication sans fil idoine. Le plus souvent, cependant, elles se basent sur un réseau local à faible consommation, typiquement Bluetooth^{®} LE, pour se connecter à un smartphone ou à un autre dispositif faisant office de passerelle.

Malgré tous les efforts mis en oeuvre pour limiter la consommation des montres connectées. La capacité des batteries reste un facteur limitant de leur utilisation. L'autonomie entre deux recharges de la batterie est souvent limitée à quelques jours ou, lorsque le GPS ou d'autres fonctions sont fortement sollicités, quelques heures.

On connaît aussi, par exemple par EP0295744, EP0547083, EP1239349, EP1821163 et autres publications, des montres électroniques qui dérivent l'énergie nécessaire à leur fonctionnement d'une génératrice électrique entraînée par les mouvements du porteur.

US 2016/252883 A1 divulgue une montre électronique avec une batterie secondaire. US 2018/0004169 A1 décrit une montre de poignet avec un récepteur GPS, EP 3190469 A1 divulgue une montre électronique capable d'effacer et afficher automatiquement son affichage numérique suivant le regard du porteur.

### Bref résumé de l'invention

Un but de la présente invention est de proposer une montre connectée dont l'utilisation n'est pas aussi limitée par la capacité des batteries que dans les dispositifs connus.

Selon l'invention, ces buts sont atteints notamment au moyen de l'objet de la revendication indépendante, des variantes préférées de réalisation étant couvertes par les revendications dépendantes.

### Brève description des figures

Des exemples de mise en oeuvre de l'invention sont indiqués dans la description illustrée par les figures annexées dans lesquelles :
- La figure 1 illustre la structure possible d'une montre selon l'invention.
- La figure 2 illustre, par un schéma-blocs, les éléments fonctionnels possibles d'une montre selon l'invention et leurs interactions.

### Exemple(s) de mode de réalisation de l'invention

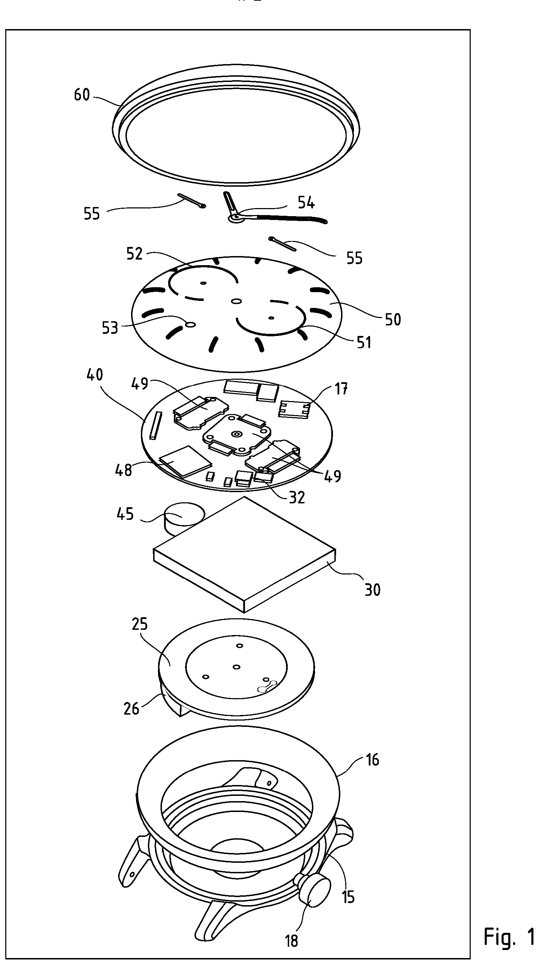

Faisant référence à la figure 1, un mode de réalisation possible de l'invention consiste en une montre de poignet comprenant un cadran analogique 50 avec deux aiguilles au centre 54, ainsi que deux petits indicateurs analogiques, par exemple un indicateur 51 à trois heures et un indicateur 52 à neuf heures, avec deux petites aiguilles 55.

La fonction principale des aiguilles au centre 54 est celle, conventionnelle, d'indiquer l'heure qu'il est. On peut envisager aussi l'utilisation des aiguilles au centre pour d'autres indications, dans le cadre de l'invention.

Les petits indicateurs 51, 52 sont utilisables pour n'importe quelle fonction. Dans une variante préférée un indicateur, par exemple l'indicateur 52, est utilisé pour indiquer à tout instant la réserve d'énergie disponible qui est liée, comme on le verra mieux par la suite, à l'état de charge de la batterie de la montre, tandis que l'autre indicateur, par exemple l'indicateur 51 peut être utilisé pour indiquer toute sorte d'information. Préférablement, un des petits indicateurs affiche un "biofeedback" correspondant à une activité physique cumulée dans un intervalle de temps déterminé, relativement à un but donné.

Pour fixer les idées l'indicateur 51 peut être programmé pour indiquer une activité physique déterminée par un ou plusieurs capteurs de mouvement et/ou d'activité physique 17. L'invention peut inclure toute sorte de capteurs comprenant par exemple un compteur de pas, un accéléromètre, un récepteur GPS, ou un cardiofréquencemètre.

Préférablement, les capteurs 17 sont compris dans la montre elle-même mais l'invention se rapporte aussi à des variantes dans lesquelles les capteurs de mouvement sont intégrés dans un dispositif externe avec lequel la montre est en communication, par exemple un téléphone portable, ou une ceinture thoracique.

Le cadrant comprend préférablement aussi une ou plusieurs diodes DEL 53 ou des éléments utilisables pour afficher une alarme visuelle. Ces éléments peuvent être activés pour notifier des situations prédéterminées à l'utilisateur comme par exemple l'arrivée d'un message ou un rendez-vous préalablement enregistré. L'alarme visuelle peut, selon les cas comporter aussi une signalisation sonore et/ou une vibration, par le vibreur 45.

La montre de l'invention comporte aussi un module électronique 40, par exemple sous forme de circuit imprimé, comprenant les capteurs 17 susmentionnés, des moteurs pas-à-pas 49 pour actionner les aiguilles 54, 55, et un ou plusieurs circuits logiques pour contrôler la montre et réaliser ses différentes fonctions.

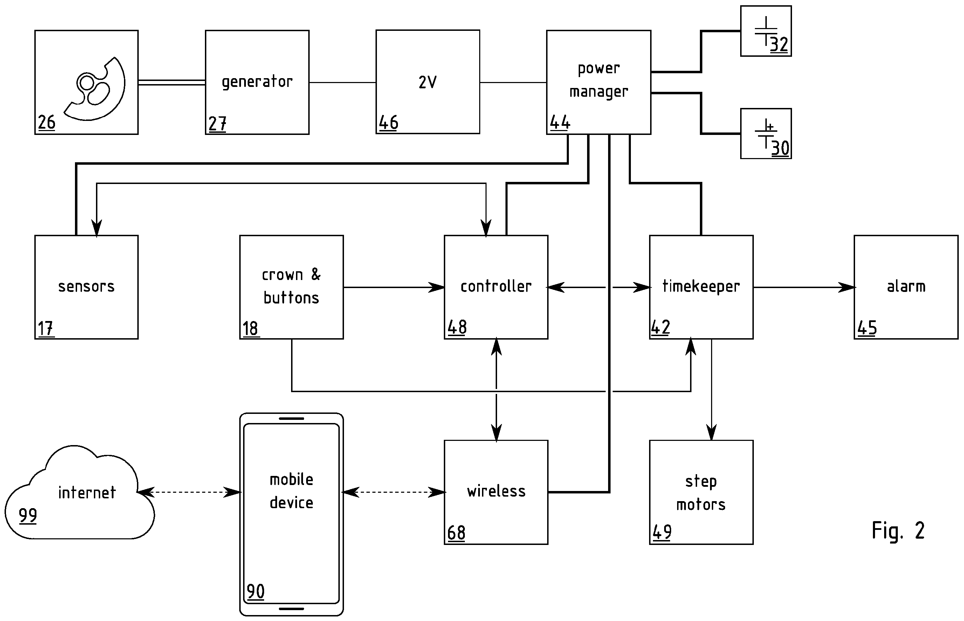

L'énergie électrique nécessaire au fonctionnement de la montre est pourvue par une unité de récolte d'énergie 25. Dans une variante préférée, l'unité de récolte d'énergie 25 comporte une masse oscillante excentrique 26 mue par les mouvements du porteur de la montre, à l'instar de la masse oscillante d'un mouvement horloger automatique, et une génératrice électrique 27 (illustrée schématiquement sur la figure 2) pour transformer ces mouvements en énergie électrique.

Préférablement, le module générateur comprend un ressort, tendu par le mouvement de la masse oscillante, et un dispositif automatique d'embrayage, pour actionner la génératrice lorsque la tension du ressort dépasse un seuil prédéterminé. Une fois le ressort détendu, la génératrice s'arrête et ce cycle, qui peut se répéter plusieurs fois par minute si le porteur est très actif, recommence.

Cette variante est avantageuse car elle permet de faire tourner la génératrice très rapidement, réduisant ainsi une partie des déperditions d'énergie qui ont lieu si elle tourne trop lentement. On peut toutefois entraîner directement la génératrice par la masse oscillante, avec une transmission synchrone avec un rapport de réduction opportun, toujours en prise. Des dispositifs de récupération d'énergie idoines sont produits par la société Kinetron (Tilburg - The Netherlands), parmi d'autres.

L'invention consent aussi des variantes à remontage manuel dans lesquelles l'unité de récolte d'énergie est entraînable par une couronne de la montre, ou par un autre élément de la montre sur lequel l'utilisateur peut agir, par exemple une lunette. La possibilité d'entraîner l'unité de récolte d'énergie 25 manuellement n'exclut pas la présence, dans la même montre, d'une masse oscillante pour réaliser la même fonction automatiquement.

La figure 2 illustre schématiquement les éléments impliqués dans le fonctionnement d'une montre selon l'invention. Cette représentation est proposée uniquement dans un but descriptif et il ne faut pas comprendre que tous les éléments dessinés sont essentiels ni qu'ils doivent être physiquement réalisés en tant qu'éléments distincts. S'agissant de blocs fonctionnels, plusieurs fonctions pourraient être assurées par un seul dispositif multifonctionnel, ou par des éléments de logiciel, selon les cas.

De manière importante la monte comporte un circuit superviseur d'alimentation 44 dont la fonction est de transformer, par exemple, le courant alternatif produit par la génératrice en courant continu adapté pour la charge d'accumulateurs, et d'emmagasiner cette énergie. Dans bien de cas, la production d'énergie électrique par la génératrice 27 est discontinue ; avantageusement le superviseur d'alimentation 44 utilise un ou plusieurs condensateurs ou supercondensateurs 32 pour faire face aux pics de production ainsi qu'une batterie rechargeable 30, par exemple un accumulateur Li-ion, Li-Poly, ou NiMH.

Une autre fonction du chargeur est celle de fournir une alimentation stable pour les autres composantes électroniques de la montre. Avantageusement, le circuit chargeur comporte un multiplicateur de tension 46 pour élever le niveau de tension présente à la sortie de la génératrice 27. Plusieurs circuits peuvent être employés à cette fin, par exemple des doubleurs à diodes ou de pompes de charge.

De manière importante, la montre de l'invention comporte deux unités logiques qui peuvent être activées de façon sélective, selon la disponibilité instantanée d'énergie. Une première unité logique 42 a la fonction de garde-temps et peut être un mouvement horloger électronique à quartz. Le garde-temps 42 pilote entre autres les moteurs pas-à-pas 49 qui actionnent les aiguilles 54, 55. Cette composante de la montre est réalisée avec des circuits intégrés à consommation ultra-faible spécialement conçus pour les applications horlogères.

Une seconde unité logique 48 est en charge des fonctions "smart" de la monte. Cette unité pourrait être réalisée par un microcontrôleur à consommation ultra-faible, ou par toute autre composante idoine. Les performances et ressources de calcul de cette seconde unité logique sont largement supérieures à celles de la première unité garde-temps 42 et sa consommation en énergie est naturellement plus importante. Préférablement, la seconde unité logique est en charge de la gestion des capteurs 17 et de l'interface de communication sans fils 68, et est active, et active les périphériques qu'elle a en gestion, selon un programme défini et la disponibilité instantanée d'énergie.

L'interface de communication sans fil 68 permet de relier la montre de l'invention à n'importe quel service ou source de données disponible sur internet. Bien qu'on pourrait envisager une connexion directe, une interface de réseau local à faible consommation, comme par exemple Bluetooth^{®} LE, est préférable pour des raisons énergétiques. Un téléphone ou un autre dispositif mobile 90 doté d'une connexion à internet est alors utilisé comme intermédiaire de communication. Le téléphone 90 est aussi employé, par une application spécifique, pour contrôler les fonctions de la montre et choisir les informations à afficher.

Préférablement, la monte de l'invention a plusieurs modes de fonctionnement qui diffèrent pour le nombre de fonctions activées, la richesse des informations affichées, et aussi par leur consommation énergétique.

La fonction de base de la montre, celle d'afficher l'heure qu'il est, est confiée entièrement au premier circuit logique garde-temps 42 et ne nécessite pas l'activation du deuxième circuit logique 48, des capteurs 17, ou de l'interface sans fils 68. La montre de l'invention comporte ainsi un mode de fonctionnement à consommation minimale, le mode "temps" dans lequel l'heure est affichée par les aiguilles 54 est actif et les fonctions "smart" sont inactives. La batterie rechargeable 30 est préférablement dimensionnée en sorte que l'autonomie de la montre en cet état est de plusieurs jours, préférablement supérieure à 100 jours. Cette autonomie correspond au temps maximal pendant lequel, en partant d'un état de charge complète de la batterie, la montre peut garder l'heure sans être portée.

Lorsque la montre est portée l'énergie produite par la génératrice 27 permet de recharger la batterie et activer des fonctions supplémentaires liées aux capteurs 17 et/ou aux informations provenant d'internet par l'interface sans fils 69. Ces fonctions peuvent inclure, par exemple :
a. monitorage du niveau d'activité physique et/ou de l'état de sommeil/éveil par un ou plusieurs capteurs, par exemple un accéléromètre un capteur de vibrations, ou un capteur de mouvement ;
b. monitorage cardiaque par un capteur optique ou électrique;
c. suivi de la position géographique par un récepteur GPS/Galileo ou tout autre système de positionnement
d. affichage de notifications déclenchées par des événements d'internet, par exemple des emails, messages de texte, ou rendez-vous.

Ces fonctions peuvent être combinées en plusieurs modes de fonctionnement, par exemple :
1. le mode "temps" avec autonomie maximale susmentionné;
2. mode "activité" dans lequel la montre détermine et stocke périodiquement, avec une cadence prédéterminée, le niveau d'activité physique, et/ou l'état d'éveil ;
3. un premier mode "sport", dans lequel la montre, en plus de l'activité physique, détermine et stocke aussi la fréquence cardiaque, par exemple une fois par minute;
4. un deuxième mode "sport", avec monitorage et enregistrement simultané de l'activité et de la position géographique.
5. un troisième mode "sport" combinant les fonctions des modes sport I et II.
6. un mode "connecté" avec monitorage de l'activité physique et affichage des notifications.
7. Dans le mode "connecté" la montre peut aussi recevoir et transmettre par l'interface sans fils 68 toute sorte de données qui ne résultent pas en une notification mais sont utiles a fonctionnement de la montre et/ou à l'implémentation de fonctions "intelligentes". Par exemple la montre peut recevoir des mises à jour de son logiciel embarqué, se synchroniser sur de serveurs de temps pour se remettre à l'heure et/ou ajuster sa marche, télécharger des données d'assistance à la géolocalisation (aGPS), et ainsi de suite.

Les modes de fonctionnement 1-6 peuvent être sélectionnés par une action délibérée de l'utilisateur, par exemple une pression sur la couronne 18 ou sur un bouton-poussoir (non représenté) ou par une app exécutée sur le dispositif mobile 90.

Préférablement, la montre de l'invention est programmée pour passer automatiquement à un mode de fonctionnement à moindre consommation en fonction de l'état de charge de la batterie 30 et des condensateurs 32, et de la puissance électrique fournie par la génératrice 27. De cette façon, si l'énergie générée par le porteur est insuffisante pour maintenir un mode activé indéfiniment, la montre passe à un mode de moindre consommation, par exemple le mode "temps" automatiquement, en sorte que l'affichage de l'heure est maintenu.

Dans une variante, le mode de fonctionnement à moindre consommation prévoit que l'affichage analogique de l'heure - par exemple les deux aiguilles 54 au centre qui indiquent les heures et les minutes - est stoppé lorsque la montre est immobile pour une période de temps plus ou moins prolongé, et reprend lorsque la montre est en mouvement. A la reprise de l'affichage les aiguilles des heures et des minutes se déplacent rapidement ("trottent") jusqu'à atteindre la position correspondante à l'heure qu'il est, telle qu'elle a été maintenue par le circuit logique garde-temps 42.

Comme on l'a vu plus haut, la petite aiguille à neuf heures est utilisée de préférence pour afficher l'état de charge de la batterie 30, tandis que l'autre donne une indication de "biofeedback" correspondant à une activité physique cumulée dans un intervalle de temps déterminé, relativement à un but donné. Cette fonction peut comporter par exemple les étapes suivantes :
a. L'utilisateur définit, par une app exécutée sur le dispositif mobile 90, un objectif d'activité physique et un intervalle de temps pendant lequel l'activité doit être complétée : par exemple 10 000 pas dans les 24 heures.
b. Le dispositif mobile 90 transfère ces consignes au processeur 48 de la montre par l'interface Bluetooth^{®} 68.
c. Le processeur 48 active un mode de fonctionnement dans lequel la fonction comptage des pas est active, par exemple le mode "activité" ci-dessus. Les pas du porteur sont comptabilisés et accumulés dans une variable.
d. Le processeur 48 calcule périodiquement le pourcentage d'activité relativement au but à atteindre et commande (éventuellement par l'action intermédiaire du mouvement horloger 42) le déplacement de la petite aiguille à trois heures en conséquence. Par exemple, l'aiguille se déplacera au milieu de sa course lorsque le porteur aura accumulé 5 000 pas, et atteindra la limite supérieure de sa course si et quand le porteur aura accumulé 10 000 pas.
e. A la fin d'une période de 24 heures la variable accumulateur est remise à zéro, et l'aiguille déplacée au début de l'échelle. Le cycle se répète chaque 24 heures.

### Numéros de référence employés sur les figures

- 15: fond
- 16: carrure
- 17: capteurs de mouvement et/ou d'activité physique
- 18: couronne
- 25: récolte d'énergie
- 26: source d'énergie
- 27: génératrice
- 30: batterie
- 32: condensateurs
- 40: circuit imprimé
- 42: unité logique garde-temps
- 44: superviseur d'alimentation
- 45: vibreur
- 46: multiplicateur de tension
- 48: microcontrôleur
- 49: moteurs pas-à-pas
- 50: cadran
- 51: indicateur fonction
- 52: indicateur réserve d'énergie
- 53: alarme visuelle
- 54: aiguilles au centre
- 55: petite aiguille
- 60: glace
- 68: interface sans fil
- 90: téléphone portable
- 99: internet

## Revendications

1. Montre de poignet électronique, comprenant un dispositif de récolte d'énergie mécanique (25) arrangé pour transformer une énergie mécanique dérivant des mouvements d'un porteur en énergie électrique, un processeur (48) en communication avec un dispositif externe (90) ou avec internet (99) par l'intermédiaire d'une interface sans fil (68) de la montre, comprenant un organe d'affichage (51) pilotable par le processeur (48) pour fournir une indication de biofeedback correspondant à une activité physique cumulée dans un intervalle de temps déterminé relativement à un but prédéterminé, un ou plusieurs capteurs (17), une unité logique (42) arrangée pour piloter un affichage (50, 54) de l'heure, un circuit superviseur d'alimentation pour stocker l'énergie électrique dans une batterie (30) et/ou dans un condensateur (32), et pour alimenter avec une énergie stockée dans la batterie (30) et/ou dans le condensateur (32) l'unité logique (42), le processeur (48), et l'interface sans fil (68), ladite montre comprenant un mode de fonctionnement à faible consommation avec fonction de garde-temps dans lequel l'unité logique (42) est active, tandis que le processeur (48), les capteurs (17), et l'interface sans fil (68) sont désactivés.

2. Montre selon l'une des revendications précédentes, dans laquelle un ou plusieurs capteurs (17) sont alimentés en énergie par le circuit superviseur (44), les capteurs (17) comprenant un ou plusieurs parmi : capteur d'activité physique, accéléromètre, récepteur pour la géolocalisation satellitaire, cardiofréquencemètre.

3. Montre selon l'une des revendications précédentes, dans laquelle le module de récolte d'énergie mécanique (25) comporte une masse oscillante (26), et/ou une couronne, et/ou une lunette, entraînant une génératrice (27).

4. Montre selon l'une des revendications précédentes, comprenant un organe d'affichage (52) pilotable par le processeur (48) pour fournir une indication de l'énergie stockée.

5. Montre selon l'une des revendications précédentes, comprenant un organe d'affichage (53) pilotable par le processeur (48) pour fournir au porteur une notification déclenchée par des événements d'internet.

6. Montre selon l'une des revendications précédentes dans laquelle l'affichage de l'heure est analogique.

7. Montre selon l'une des revendications précédentes, ledit processeur (48) étant programmé pour avoir plusieurs modes de fonctionnement ayant des consommations énergétiques différentes, et pour sélectionner automatiquement un mode de fonctionnement à consommation inférieure en fonction de l'état de charge de la batterie (30) et/ou des condensateurs (32), et de la puissance électrique fournie par le système de récolte d'énergie (25).

8. Montre selon la revendication précédente, dans laquelle un affichage analogique de l'heure est stoppé lorsque la montre est immobile et dans le mode de fonctionnement à faible consommation et redémarré lorsque la montre est en mouvement.

9. Montre selon la revendication précédente, comprenant une batterie (30) et/ou un condensateur (32) dimensionnés pour assurer une autonomie supérieure à 100 jours dans le mode à faible consommation, l'autonomie correspondant au temps maximal pendant lequel, en partant d'un état de charge complète de la batterie et/ou du condensateur, la montre peut garder l'heure sans être portée.

10. Montre selon l'une des revendications de 3 à 12, la montre étant arrangé pour transmettre des informations obtenues des capteurs à un dispositif électronique (90) connectable avec l'interface sans fil de la montre, les informations obtenues par les capteurs pouvant comprendre, entre autres, une ou plusieurs parmi : activité physique du porteur de la montre, position géographique, fréquence cardiaque ou vasculaire.

11. Système comprenant la montre selon la revendication précédente en combinaison avec un dispositif connectable avec l'interface sans fil de la montre, notamment un téléphone (90), le dispositif étant programmé pour afficher les informations obtenues, par exemple sous forme d'un indicateur d'activité, ou d'une indication de biofeedback correspondant à une activité physique cumulée dans un intervalle de temps déterminé.

## Patentansprüche

1. Elektronische Armbanduhr, umfassend eine mechanische Energiegewinnungsvorrichtung (25), welche dazu eingerichtet ist, mechanische Energie, die aus den Bewegungen eines Trägers stammt, in elektrische Energie umzuwandeln, einen Prozessor (48), der über eine drahtlose Schnittstelle (68) der Uhr mit einem externen Gerät (90) oder mit dem Internet (99) kommuniziert, umfassend ein Anzeigeelement (51), welches vom Prozessor (48) steuerbar ist, um eine Biofeedbackangabe entsprechend der kumulativen körperlichen Aktivität in einem bestimmten Zeitintervall bereitzustellen, einen oder mehrere Sensoren (17), eine Logikeinheit (42) zur Steuerung einer Zeitanzeige (50, 54), eine Stromversorgungsüberwachungsschaltung (44) zum Speichern von elektrischer Energie in einer Batterie (30) und/oder in einem Kondensator (32) und zur Versorgung der Logikeinheit (42), des Prozessors (48) und der drahtlosen Schnittstelle (68) mit der in der Batterie (30) und/oder im Kondensator (32) gespeicherten Energie, wobei die besagte Uhr einen verbrauchsarmen Betriebsmodus mit Zeitnehmerfunktion umfasst, worin die Logikeinheit (42) aktiv ist, während der Prozessor (48), die Sensoren (17) und die drahtlose Schnittstelle (68) deaktiviert sind.

2. Uhr gemäss einem der vorhergehenden Ansprüche, worin eine oder mehrere Sensoren (17) von der Überwachungsschaltung (44) mit Energie versorgt werden, wobei die Sensoren (17) einen oder mehrere von Folgendem umfassen: Sensor für körperliche Aktivität, Beschleunigungsmesser, Empfänger für Satellitengeolokalisierung, Herzfrequenzmessgerät.

3. Uhr gemäss einem der vorhergehenden Ansprüche, worin das mechanische Energiegewinnungsmodul (25) eine oszillierende Masse (26) und/oder eine Krone und/oder eine Lünette umfasst, die einen Generator (27) antreibt.

4. Uhr gemäss einem der vorhergehenden Ansprüche, mit einem vom Prozessor (48) steuerbaren Anzeigeelement (52), um eine Anzeige der gespeicherten Energie bereitzustellen.

5. Uhr gemäss einem der vorhergehenden Ansprüche, mit einem vom Prozessor (48) steuerbares Anzeigeelement (53), um dem Träger eine durch Internetereignisse ausgelöste Benachrichtigung bereitzustellen.

6. Uhr gemäss einem der vorhergehenden Ansprüche, worin die Zeitanzeige analog ist.

7. Uhr gemäss einem der vorhergehenden Ansprüche, wobei der Prozessor (48) derart programmiert ist, dass er über mehrere Betriebsmodi mit unterschiedlichem Energieverbrauch verfügt und je nach Ladezustand der Batterie (30) und/oder Kondensatoren (32) und je nach vom Energiegewinnungssystem (25) gelieferten elektrischen Energie automatisch einen Betriebsmodus mit geringerem Verbrauch auswählt.

8. Uhr gemäss dem vorhergehenden Anspruch, worin eine analoge Zeitanzeige bei Stillstand der Uhr und im verbrauchsarmen Betriebsmodus gestoppt und bei Bewegung der Uhr neu gestartet wird.

9. Uhr gemäss dem vorhergehenden Anspruch, mit einer Batterie (30) und/oder einem Kondensator (32), welche so dimensioniert sind, dass sie eine Autonomie von mehr als 100 Tagen im Niedrigverbrauchsmodus gewährleisten, wobei die Autonomie der maximalen Zeit entspricht, während der, ausgehend von einem vollständig aufgeladenen Zustand der Batterie und/oder des Kondensators, die Uhr die Zeit anzuzeigen vermag, ohne dass sie getragen werden muss.

10. Uhr gemäss einem der Ansprüche 3 bis 9, wobei die Uhr dazu eingerichtet ist, von den Sensoren (17) erhaltene Informationen an ein elektronisches Gerät (90) zu übertragen, das mit der drahtlosen Schnittstelle der Uhr verbunden werden kann, wobei die von den Sensoren erhaltenen Informationen unter anderem einen oder mehrere von Folgendem umfassen können: körperliche Aktivität des Trägers der Uhr, geografische Position, Herz- oder Gefässfrequenz.

11. System mit dem Uhr gemäss dem vorhergehenden Anspruch in Kombination mit einem mit der drahtlosen Schnittstelle der Uhr verbindbaren Gerät, insbesondere einem Telefon (90), wobei das Gerät derart programmiert ist, dass es die erhaltenen Informationen anzeigt, beispielsweise in Form einer Aktivitätsanzeige, oder einer Biofeedback-Anzeige entsprechend der kumulativen körperlichen Aktivität in einem bestimmten Zeitintervall.

## Claims

1. Electronic wristwatch, comprising a mechanical energy harvester device (25), designed to transform mechanical energy deriving from the movements of a wearer to electrical energy, a processor (48) in communication with an external device (90) or with internet (99) through a wireless interface (68) of the watch, comprising a display component (51), controllable by the processor (48), for providing a biofeedback indication corresponding to a physical activity accumulated in a determined time interval relative to a predetermined goal, one or several sensors (17), a logic unit (42) designed to control a display (50, 54) of the time, a power manager circuit (44) for storing the electrical energy in a battery (30) and/or in a capacitor (32), and to supply the logic unit (42), the processor (48) and the wireless interface (68) with energy stored in the battery (30) and/or in the capacitor (32), said watch comprising having a low consumption functioning mode with timekeeping function in which the logic unit (42) is active, while the processor (48), the sensors (17) and the wireless interface (68) are deactivated.

2. Watch according to one of the preceding claims, wherein one or more sensors (17) are supplied in energy by the power manager (44), the sensors (17) comprising one or more among: physical activity sensor, accelerometer, receiver for satellite geopositioning, heart rate monitor.

3. Watch according to one of the preceding claims, wherein the mechanical energy harvester module (25) comprises an oscillating weight (26) and/or a crown, and/or a bezel, driving a generator (27).

4. Watch according to one of the preceding claims, comprising a display component (52), controllable by the processor (48), for providing an energy reserve indication.

5. Watch according to one of the preceding claims, comprising a display component (53), controllable by the processor (48), for providing the wearer with a notification triggered by internet events.

6. Watch according to one of the preceding claims, wherein the time display is analog.

7. Watch according to one of the preceding claims, said processor (48) being programmed to have several functioning modes having different energy consumptions, and to select automatically a lower consumption functioning mode according to the state of charge of the battery (30) and/or the capacitors (32), and the electric power supplied by the energy harvester system (25).

8. Watch according to the preceding claim, wherein an analog display of the time is stopped when the watch is immobile and in the low consumption functioning mode and started again when the watch is moving.

9. Watch according to the preceding claim, comprising a battery (30) and/or a capacitor (32) dimensioned to ensure an autonomy longer than 100 days in the low consumption mode, the autonomy corresponding to the maximum time during which, starting from a full state of charge of the battery and/or of the capacitor, the watch can keep time without being worn.

10. Watch according to one of the claims 3 to 9, the watch being designed to transmit information obtained from the sensors (17) to an electronic device (90) connectable to the wireless interface of the watch, wherein the information received by the sensors can comprise, among others, one or more among: physical activity of the wearer of the watch, geographical position, heart rate or vascular rhythm.

11. System comprising the watch according to the preceding claim in combination with a device connectable to the wireless interface of the watch, in particular a telephone (90), the device being programmed to display the information obtained, for example in the form of an activity indicator, or a biofeedback indication corresponding to a physical activity accumulated in a determined time interval.
